# EUROPEAN PATENT APPLICATION

(11) **EP 4 656 725 A1**
(43) Date of publication of application: **03.12.2025**
(21) Application number: 24747426.5
(22) Date of filing: 23.01.2024
(51) Int. Cl.: C12N 15/79, C12N 15/87, C07K 14/405, C12N 9/24, C12R 1/89

(54) **COMPOSITION FOR LEACHING LITHIUM, NICKEL OR COBALT USING CHLORELLA VULGARIS STRAIN AND METHOD FOR LEACHING LITHIUM, NICKEL OR COBALT**

(30) Priority: 25.01.2023 KR 20230009737
(71) Applicant: Green Mineral Inc., Seoul (KR); Sogang University Research Business Development Foundation, Seoul 04107 (KR)
(72) Inventor: JUNG, Kwang-Hwan, Seongnam-si, Gyeonggi-do 13566 (KR); SHIM, Jingon, Seoul 03970 (KR); LEE, Hosuk Sean, Seoul 03113 (KR)
(74) Representative: Reddie & Grose LLP
(86) International application number: PCT/KR2024/001067
(87) International publication number: WO 2024/158190

(57) **Abstract**

The present invention relates to a composition for leaching lithium, nickel or cobalt, and a method for leaching lithium, nickel or cobalt, using a *Chlorella vulgaris* strain.

## Description

### TECHNICAL FIELD

The present patent application claims the priority based on Korean Patent Application No. 10-2023-0009737 filed on January 25, 2023, and the disclosure of the patent application is incorporated in the present description by reference.

The present invention relates to a composition for leaching lithium, nickel or cobalt, and a method for leaching lithium, nickel or cobalt, using a *Chlorella vulgaris* strain.

### BACKGROUND ART

*Chlorella vulgaris* is a unicellular microalga belonging to the Chlorophyta phylum. *Chlorella vulgaris* possesses photosynthetic apparatus and while performing photosynthesis, it can grow rapidly using only light, carbon dioxide, water, and a small amount of minerals, so it is widely used in studies for mass production of useful substances (photobioreactor).

In addition, since the biomass has a relatively high glycolipid content of about 42%, it has also been actively used in research for developing biofuels that can be used as an alternative to biodiesel. Recently, studies have also reported that *Chlorella vulgaris* can be used to remove a radioactive isotope, ⁹⁰Sr.

As a result of electroporation using a hygromycin B resistance gene, a zeocin resistance gene, and a chloramphenicol acetyltransferase gene (CAT gene) and the like as a selectable marker, although integration into chromosomal DNA occurred, transformants were temporarily formed and soon lost the resistance.

As a transformation method, glass beads, electroporation, and agrobacterium-mediated transformation and the like have been used, but due to the lack of an efficient selectable marker and transformation system, the use of *Chlorella vulgaris* remains limited.

With the development of technology, the amount of electricity used is rapidly increasing, and the efficiency of energy consumption and production is also improving. The amount of electricity used is rapidly increasing, and for efficiency in production according thereto, the world is focusing on production of electricity using nuclear power plants. A problem that is always raised along with efficiency of production thereof, is right a problem that the risk posed by nuclear power plant accidents and radioactive contamination in contaminated water and contamination caused by metal ions dose not disappear.

In addition, rare metals such as Sr, Cs, Li, Ni, Co, and the like which are used in industrial fields, face a national issue of dependency on overseas imports, and as the market for secondary batteries grows, this issue becomes more pronounced, and the problem of recycling is emerging as a significant industrial and national issue.

In order to recover metals from ore or secondary resources (waste catalysts or waste electronic devices), hydrometallurgical or pyrometallurgical methods are mainly used. However, hydrometallurgy using acid and alkaline leaching methods results in severe environmental pollution due to the use of strong acid and strong alkaline solvents during metal leaching, and in the case of pyrometallurgy, a large amount of sulfide gas is generated, causing many problems in facility operation. Accordingly, interest is increasing in bioleaching, which is a more environmentally friendly leaching method.

Bioleaching is a type of hydrometallurgical refining method that recover valuable metals using a microorganism capable of leaching metals. However, bioleaching, known to be environmentally friendly, has the drawback of low efficiency of metal leaching. Accordingly, there is an urgent need to develop a bioleaching technology that has high efficiency of metal leaching and is environmentally friendly.

### DISCLOSURE

### TECHNICAL PROBLEM

Accordingly, the present inventors have confirmed that a *Chlorella vulgaris* strain can leach lithium, nickel or cobalt from a substance containing lithium, nickel or cobalt with excellent efficiency, and in particular, it was confirmed that the *Chlorella vulgaris* strain transformed with the vector of the present invention exhibited even more excellent efficiency of leaching lithium, nickel or cobalt, thereby completing the present invention.

The present inventors predicted promoter and terminator sites of *Coccomyxa* C-169 (hereinafter, C-169; previously known as *Chlorella vulgaris,* but renamed) and obtained sequences, and synthesized them by fusion with the *Sh ble* gene. Any intron was not inserted into the middle of the *Streptomyces verticillus* bleomycin (*Sh ble*) gene, and the synthesized DNA fragment was treated with SwaI/KpnI restriction enzymes and cloned into the pSP124S vector, and this was named pKA650.

In addition, the target gene, beta-type carbonic anhydrase, was cloned into the middle of the *Streptomyces verticillus* bleomycin (*Sh ble*) gene into the pKa650 vector, and this was named pJG002.

Then, after transformation by gold particle bombardment, it was cultured in a medium containing zeocin and was selectively selected. Colonies were obtained by culturing through selection, thereby producing transformants.

In addition, it was confirmed that lithium, nickel or cobalt can be leached with excellent efficiency from a substance containing lithium, nickel or cobalt using *Chlorella vulgaris* or a transformant thereof.

Accordingly, an object of the present invention is to provide a composition for leaching lithium, nickel or cobalt, comprising at least one kind selected from the group consisting of a *Chlorella vulgaris* strain, a culture of the strain, a concentrate of the culture, and a dried product of the culture.

Another object of the present invention is to provide a method for leaching lithium, nickel or cobalt through bioleaching, comprising a mixing step of mixing at least one kind selected from the group consisting of a *Chlorella vulgaris* strain, a culture of the strain, a concentrate of the culture, and a dried product of the culture, with a substance containing lithium, and a leaching step of leaching lithium for a predetermined period of time.

Other object of the present invention is to provide a use of a *Chlorella vulgaris* strain for leaching lithium, nickel or cobalt.

### TECHNICAL SOLUTION

The present invention relates to a composition for leaching lithium, nickel or cobalt, and a method for leaching lithium, nickel or cobalt, using a *Chlorella vulgaris* strain.

Hereinafter, the present invention will be described in more detail.

One aspect of the present invention relates to a composition for leaching lithium, nickel or cobalt, comprising at least one kind selected from the group consisting of a *Chlorella vulgaris* strain, a culture of the strain, a concentrate of the culture, and a dried product of the culture.

In the present invention, the *Chlorella vulgaris* strain can leach lithium, nickel or cobalt by crystallizing lithium, nickel or cobalt from a substance comprising lithium, nickel or cobalt through biomineralization.

The term "leaching" in the present description refers to separation of a target component, and may be interchangeably used with "separating" , "extracting" or "eluting."

The term "composition for leaching" in the present description refers to a composition for separation of a target component, and may be interchangeably used with "composition for biomineralization" or "composition for bioleaching."

In the present invention, the strain may be transformed with a vector comprising the following:
a promoter of the *Coccomyxa* C-169 ribulose-1,5-bisphosphate carboxylase/oxygenase (RuBisCo) small subunit 2 (rbcS2) gene;
a nucleotide sequence encoding beta-type carbonic anhydrase of Coccomyxa subellipsoidea C-169; and
a terminator sequence of the *Coccomyxa* C-169 rbcS2 gene.

In the present invention, the promoter may comprise the nucleotide sequence of SEQ ID NO: 2, or may comprise a nucleotide sequence with substantial identity to the nucleotide sequence of SEQ ID NO: 2, and for example, it may consist of the nucleotide sequence of SEQ ID NO: 2, but not limited thereto.

In one embodiment of the present invention, the promoter comprising the nucleotide sequence of SEQ ID NO: 2 may be a sequence further comprising a base of 1 to 50 bp at the 3' end of the sequence of SEQ ID NO: 2 comprised in SEQ ID NO: 1, but not limited thereto.

In the present invention, the terminator sequence may comprise the nucleotide sequence of SEQ ID NO: 3, or may comprise a nucleotide sequence with substantial identity to the nucleotide sequence of SEQ ID NO: 3, and for example, it may consist of the nucleotide sequence of SEQ ID NO: 3, but not limited thereto.

In the present invention, a target protein-encoding nucleotide sequence may be operatively linked to a promoter.

In the present invention, the term "operatively linked" refers to functional binding between a nucleic acid expression regulatory sequence (for example, a promoter sequence, a signal sequence or an array of transcription regulatory factor binding sites) and another nucleic acid sequence, and thereby, the regulatory sequence regulates transcription and/or translation of the another nucleic acid sequence.

In the present invention, the target protein-encoding nucleotide sequence may be linked to the 3' end of the terminator sequence, but not limited thereto.

In the present invention, the vector may further comprise a selectable marker.

In the present invention, the selectable market may be an antibiotic resistance gene, but not limited thereto.

In the present invention, the antibiotic may be at least one kind selected from the group consisting of spectinomycin, paromomycin, ampicillin, zeocin and bleomycin, but not limited thereto.

In the present invention, the selectable marker may be selected from various selectable marker genes against conventional antibiotics to be used. Examples thereof include aminoglycoside phosphotransferase, which confers kanamycin antibiotic resistance, chloramphenicol acetyltransferase, which is involved in chloramphenicol antibiotic resistance, and the like.

In the present invention, when the selectable marker is bleomycin, the selectable marker may comprise the nucleotide sequence of SEQ ID NO: 4, or may comprise a nucleotide sequence with substantial identity to the nucleotide sequence of SEQ ID NO: 4, and for example, it may consist of the nucleotide sequence of SEQ ID NO: 4, but not limited thereto.

In the present invention, a method for selecting transformed *Chlorella vulgaris* into which a selectable marker has been introduced may be easily conducted using a phenotype expressed by the selectable marker according to a method widely known in the art. For example, when the selectable marker is a specific antibiotic resistance gene, transformants can be easily selected by culturing transformants in a medium containing the antibiotic.

In the present invention, the vector may further comprise a gene encoding a reporter molecule.

In the present invention, the reporter molecule may be at least one kind selected from the group consisting of a growth-promoting protein, a fluorescent protein and a hydrolytic enzyme, but not limited thereto.

In the present invention, the fluorescent protein may be luciferase, but not limited thereto.

In the present invention, the hydrolytic enzyme may be β-glucuronidase, but not limited thereto.

In the present invention, the vector may comprise the nucleotide sequence of SEQ ID NO: 1, or may comprise a nucleotide sequence with substantial identity to the nucleotide sequence of SEQ ID NO: 1, and for example, it may consist of the nucleotide sequence of SEQ ID NO: 1, but not limited thereto.

In the present invention, the vector may be for microalgal transformation using gold particles bombardment.

In the present invention, "vector" refers to a means for expressing a target gene in a host cell. For example, it includes plasmid vectors, cosmid vectors, and bacteriophage vectors, and viral vectors such as adenoviral vectors, retroviral vectors and adeno-associated viral vectors.

In the present invention, the vector which can be used as a recombinant vector may be produced by modifying plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), phages (e.g., λgt4λB, λ-Charon, λ Δz1 and M13, etc.) or viruses (e.g., SV40, etc.), commonly used in the art, and for example, it may be the pSP124S vector backbone, but not limited thereto.

In the present invention, the vector may be constructed typically as a vector for cloning or a vector for expression.

In the present invention, as the vector for expression, a common one used for expressing a target protein in a plant, animal or microorganism may be used.

In the present invention, the vector may be constructed by various methods known in the art.

In the present invention, the term "substantial identity" refers to that the any other nucleotide sequence has a sequence homology of at least 70% or more, 90% or more, or 98% or more with each nucleotide sequence, by aligning a given nucleotide sequence with any other nucleotide sequence to maximize correspondence, and analyzing the sequences.
a situation where a given nucleotide sequence is aligned with any other nucleotide sequence to maximize correspondence, and upon analysis of the sequences, the other nucleotide sequence exhibits a sequence homology of at least 70%, 90%, or 98% with the given nucleotide sequence.

Another aspect of the present invention relates to a method for leaching lithium, nickel or cobalt through bioleaching, comprising a mixing step of mixing at least one kind selected from the group consisting of a *Chlorella vulgaris* strain, a culture of the strain, a concentrate of the culture, and a dried product of the culture, with a substance containing lithium, and a leaching step of leaching lithium for a predetermined period of time.

In the present invention, the method for leaching lithium, nickel or cobalt may comprise the following transforming step before the mixing step, and the *Chlorella vulgaris* strain of the mixing step may be a transformed *Chlorella vulgaris* strain:
a transforming step of introducing a vector comprising a promoter of the *Coccomyxa* C-169 ribulose-1,5-bisphosphate carboxylase/oxygenase (RuBisCo) small subunit 2 (rbcS2) gene, a nucleotide sequence encoding beta-type carbonic anhydrase of Coccomyxa subellipsoidea C-169, and a terminator sequence of the *Coccomyxa* C-169 rbcS2 gene, into *Chlorella vulgaris.*

In the present invention, the promoter may comprise the nucleotide sequence of SEQ ID NO: 2, or may comprise a nucleotide sequence with substantial identity to the nucleotide sequence of SEQ ID NO: 2, and for example, it may consist of the nucleotide sequence of SEQ ID NO: 2, but not limited thereto.

In one embodiment of the present invention, the promoter comprising the nucleotide sequence of SEQ ID NO: 2 may be a sequence further comprising a base of 1 to 50 bp at the 3' end of the sequence of SEQ ID NO: 2 comprised in SEQ ID NO: 1, but not limited thereto.

In one embodiment of the present invention, the promoter comprising the nucleotide sequence of SEQ ID NO: 2 may be a sequence further comprising a base of 1 to 50 bp at the 3' end of the sequence of SEQ ID NO: 2 comprised in SEQ ID NO: 7, but not limited thereto.

In the present invention, the terminator sequence may comprise the nucleotide sequence of SEQ ID NO: 3, or may comprise a nucleotide sequence with substantial identity to the nucleotide sequence of SEQ ID NO: 3, and for example, it may consist of the nucleotide sequence of SEQ ID NO: 3, but not limited thereto.

In the present invention, a target protein-encoding nucleotide sequence may be operatively linked to a promoter.

In the present invention, the target protein-encoding nucleotide sequence may be linked to the 3' end of the terminator sequence, but not limited thereto.

In the present invention, the vector may further comprise a selectable marker.

In the present invention, the selectable market may be an antibiotic resistance gene, but not limited thereto.

In the present invention, the antibiotic may be at least one kind selected from the group consisting of spectinomycin, paromomycin, ampicillin, zeocin and bleomycin, but not limited thereto.

In the present invention, the selectable marker may be selected from various selectable marker genes against conventional antibiotics to be used. Examples thereof include aminoglycoside phosphotransferase, which confers kanamycin antibiotic resistance, chloramphenicol acetyltransferase, which is involved in chloramphenicol antibiotic resistance, and the like.

In the present invention, when the selectable marker is bleomycin, the selectable marker may comprise the nucleotide sequence of SEQ ID NO: 4, or may comprise a nucleotide sequence with substantial identity to the nucleotide sequence of SEQ ID NO: 4, and for example, it may consist of the nucleotide sequence of SEQ ID NO: 4, but not limited thereto.

In the present invention, a method for selecting transformed *Chlorella vulgaris* into which a selectable marker has been introduced may be easily conducted using a phenotype expressed by the selectable marker according to a method widely known in the art. For example, when the selectable marker is a specific antibiotic resistance gene, transformants can be easily selected by culturing transformants in a medium containing the antibiotic.

In the present invention, the vector may further comprise a gene encoding a reporter molecule.

In the present invention, the reporter molecule may be at least one kind selected from the group consisting of a growth-promoting protein, a fluorescent protein and a hydrolytic enzyme, but not limited thereto.

In the present invention, the fluorescent protein may be luciferase, but not limited thereto.

In the present invention, the hydrolytic enzyme may be β-glucuronidase, but not limited thereto.

In the present invention, the vector may comprise the nucleotide sequence of SEQ ID NO: 1, or may comprise a nucleotide sequence with substantial identity to the nucleotide sequence of SEQ ID NO: 1, and for example, it may consist of the nucleotide sequence of SEQ ID NO: 1, but not limited thereto.

In the present invention, the vector may comprise the nucleotide sequence of SEQ ID NO: 7, or may comprise a nucleotide sequence with substantial identity to the nucleotide sequence of SEQ ID NO: 7, and for example, it may consist of the nucleotide sequence of SEQ ID NO: 7, but not limited thereto.

In the present invention, the vector may be for microalgal transformation using gold particles bombardment.

In the present invention, the vector which can be used as a recombinant vector may be produced by modifying plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), phages (e.g., λgt4λB, λ-Charon, λΔz1 and M13, etc.) or viruses (e.g., SV40, etc.), commonly used in the art, and for example, it may be the pSP124S vector backbone, but not limited thereto.

In the present invention, the vector may be constructed as a vector for cloning or a vector for expression.

In the present invention, as the vector for expression, a common one used for expressing a target protein in a plant, animal or microorganism in the art may be used.

In the present invention, the vector may be constructed by various methods known in the art.

In the present invention, the transforming step may be performed using gold particles bombardment.

In the present invention, the cell stage of the transforming step may be a log phase. When it is the log phase, it is most efficient.

In the present invention, the log phase may have an OD686 value of 0.4 to 0.6, 0.45 to 0.6, 0.5 to 0.6, 0.55 to 0.6, for example, 0.6.

In the present invention, the cell density of the transforming step may be 5.0*10⁶ to 8.0*10⁷ per 60mm Diameter, 1.0*10⁷ to 8.0*10⁷ per 60mm Diameter, 2.0*10⁷ to 8.0*10⁷ per 60mm Diameter, 3.0*10⁷ to 8.0*10⁷ per 60mm Diameter, 4.0*10⁷ to 8.0*10⁷ per 60mm Diameter, 1.0*10⁷ to 7.0*10⁷ per 60mm Diameter, 2.0*10⁷ to 7.0*10⁷ per 60mm Diameter, 3.0*10⁷ to 7.0*10⁷ per 60mm Diameter, 4.0*10⁷ to 7.0*10⁷ per 60mm Diameter, 1.0*10⁷ to 6.0*10⁷ per 60mm Diameter, 2.0*10⁷ to 6.0*10⁷ per 60mm Diameter, 3.0*10⁷ to 6.0*10⁷ per 60mm Diameter, 4.0*10⁷ to 6.0*10⁷ per 60mm Diameter, 1.0*10⁷ to 5.0*10⁷ per 60mm Diameter, 2.0*10⁷ to 5.0*10⁷ per 60mm Diameter, 3.0*10⁷ to 5.0*10⁷ per 60mm Diameter, 4.0*10⁷ to 5.0*10⁷ per 60mm Diameter, for example, 4.8*10⁷ per 60mm Diameter.

In the present invention, the vacuum of the transforming step may be 27.0 to 29.0 inchs Hg, and the vacuum may be 27.5 to 29.0 inchs Hg, 28.0 to 29.0 inchs Hg, 28.5 to 29.0 inchs Hg, for example, 29.0 inchs Hg.

In the present invention, the target distance of the transforming step may be 3 to 9, and for example, it may be 3, 6 or 9.

In the present invention, the pressure of the transforming step may be 1200 to 1300 psi, 1210 to 1300 psi, 1220 to 1300 psi, 1230 to 1300 psi, 1240 to 1300 psi, 1250 to 1300 psi, 1260 to 1300 psi, 1270 to 1300 psi, 1280 to 1300 psi, 1290 to 1300 psi, for example, 1300 psi.

In the present invention, the substance containing lithium, nickel or cobalt may be at least one selected from the group consisting of batteries containing LiCl solution, Li₂SO₄ solution, lithium, batteries containing NiCl₂ solution, NiSO₄ solution, nickel, batteries containing CoCl₂ solution, CoSO₄ solution, cobalt, waste batteries, waste water, and salt lakes, but not limited thereto.

### ADVANTAGEOUS EFFECTS

The present invention relates to a composition for leaching lithium, nickel or cobalt, and a method for leaching lithium, nickel or cobalt, using a *Chlorella vulgaris* strain.

### BRIEF DESCRIPTION OF THE DRAWINGS

FIG. 1 is the pKA650 vector map according to one example of the present invention.
FIG. 2 is a photograph showing the result confirming that transformation was inefficient and not successfully achieved when using a glass bead method according to one example of the present invention.
FIG. 3 is a photograph showing the result of introducing a resistance gene into the vector system according to one example of the present invention and transforming it to obtain mutants with antibiotic resistance.
FIG. 4 is a photograph showing the result of confirming the genetic information of the mutants obtained according to one example of the present invention.
FIG. 5 is a photograph showing the result of subculturing to confirm the maintenance of the mutants according to one example of the present invention.
FIG. 6 is the pJG002 vector map according to one example of the present invention.
FIG. 7 is a result of confirming that the antibiotic resistance is maintained by concentration, after performing biolistic transformation as Example 2, and subculturing in relation thereto according to one example of the present invention.
FIG. 8 is a photograph confirming a band size of about 43kDa of the target protein, beta-type carbonic anhydrase, through whole protein SDS-PAGE, in order to confirm the gene and protein expression of the transformants according to one example of the present invention.
FIG. 9 is a result of performing MALDI-TOF to verify the band of the target protein according to one example of the present invention.
FIG. 10 is a photograph showing the result of confirming the presence of the target gene by Southern blot for genetic verification according to one example of the present invention.
FIG. 11 is a photograph showing the result of comparing Sr biomineralization of the wild type and transformant according to one example of the present invention.
FIG. 12 is a result of analysis through ICP-MS for quantitative comparison of Sr biomineralization of the wild type and transformant according to one example of the present invention.
FIG. 13 and FIG. 14 are graphs of results of confirming the removal rate according to a single LiCl concentration according to one example of the present invention.
FIG. 15 and FIG. 16 are graphs of results of measuring the effects (by concentration: 300, 600, 900mM) on sodium and sulfate according to one example of the present invention.
FIG. 17 is a graph showing the composition of the lithium battery solution according to one example of the present invention.
FIG. 18 is a graph showing the result of Li crystallization in the lithium battery solution according to one example of the present invention.
FIG. 19 and FIG. 20 are graphs showing results of crystallization (by concentration: 300, 600, 900mM) by Na, S in the lithium battery solution according to one example of the present invention.
FIG. 21 and FIG. 22 are graphs of results of confirming the removal rate according to a single NiCl₂ concentration according to one example of the present invention.
FIG. 23 and FIG. 24 are graphs of results of measuring the effects (by concentration: 300, 600, 900mM) on sodium and sulfate according to one example of the present invention.
FIG. 25 is a graph showing the composition of the nickel battery solution according to one example of the present invention.
FIG. 26 is a graph showing the result of Ni crystallization in the nickel battery solution according to one example of the present invention.
FIG. 27 and FIG. 28 are graphs showing results of crystallization (by concentration: 300, 600, 900mM) by Na, S in the nickel battery solution according to one example of the present invention.
FIG. 29 and FIG. 30 are graphs of results of confirming the removal rate according to a single CoCl₂ concentration according to one example of the present invention.
FIG. 31 and FIG. 32 are graphs of results of measuring the effects (by concentration: 300, 600, 900mM) on sodium and sulfate according to one example of the present invention.
FIG. 33 is a graph showing the composition of the cobalt battery solution according to one example of the present invention.
FIG. 34 is a graph showing the result of Co crystallization in the cobalt battery solution according to one example of the present invention.
FIG. 35 and FIG. 36 are graphs showing results of crystallization (by concentration: 300, 600, 900mM) by Na, S in the cobalt battery solution according to one example of the present invention.

### BEST MODE

A composition for leaching lithium, nickel or cobalt, comprising at least one kind selected from the group consisting of a *Chlorella vulgaris* strain, a culture of the strain, a concentrate of the culture, and a dried product of the culture.

### MODE FOR INVENTION

Hereinafter, the present invention will be described in more detail by the following examples. However, these examples are intended to illustrate the present invention only, but the scope of the present invention is not limited by these examples.

### Preparative example 1. Plasmid construction and cloning

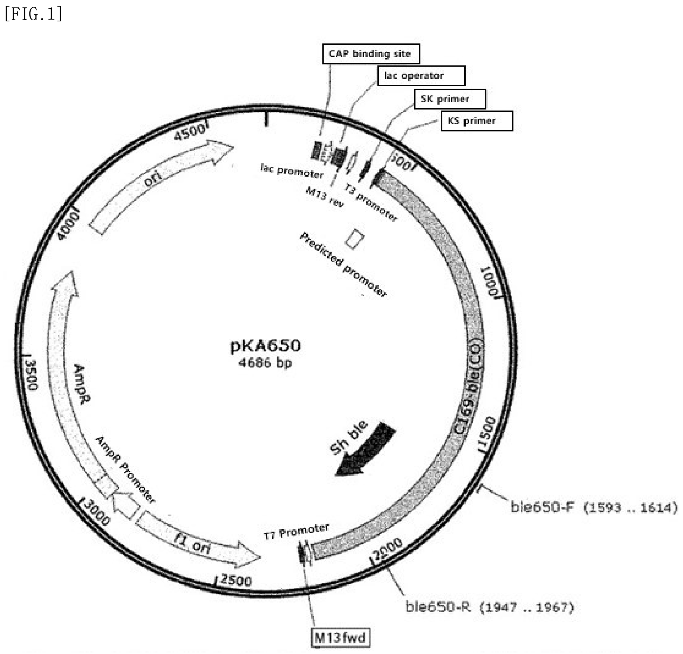

The sequence of the bleomycin resistance gene (*Sh ble*) was obtained from genomic DNA of *Streptomyces verticillus. Sh-ble,* which is the entire sequence obtained by conjugating the promoter sequence of the *Coccomyxa C-*169 *rbcS2* gene at the 5' end of the bleomycin resistance gene and the terminator sequence of the *Coccomyxa* C-169 *rbcS2* gene at the 3' end, was synthesized (GenScript, USA). The synthesized gene was inserted into the pSP124S vector by treatment with restriction enzymes Swa I and Kpn I. The obtained plasmid was named pKA650 and the vector map was shown in FIG. 1.

As can be confirmed in FIG. 1, OriV is a replication origin, and *Sh ble* is the *Sh ble* gene to which the promoter and terminator of *Streptomyces verticillus* bleomycin are linked, and AmpR is an ampicillin resistance gene), and C-169-ble is the promoter sequence of the *Coccomyxa* C-169 *rbcS2* gene, and the 3' region is the terminator site of the *Coccomyxa* C-169 *rbcS2* gene in the vector map.

### Preparative example 2. Transformation into Chlorella vulgaris

### 2-1. Transformation using a glass bead method

A Simple and Rapid Glass Bead Transformation Method was used by glass beads. This method is a method of adding glass beads and applying physical force, and through physical friction, making pores in the cell membrane of cells, to introduce a desired plasmid.

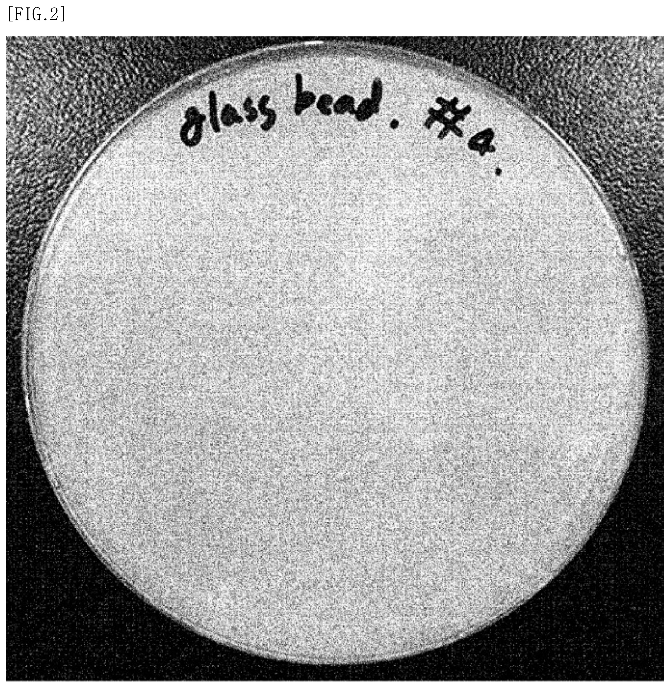

The experiment was conducted according to the conditions described in Table 1. Specifically, the cells were vortexed with glass beads at room temperature (25°C) to disrupt a part of the cells. Then, by separating the beads and supernatant by gravity, DNA was added to the supernatant. Next, the recovery was conducted by slowly rotating at 37°C. After dispensing onto solid media containing an antibiotic, transformants were identified. The results were shown in FIG. 2.

**[Table 1]**

| **Date** | **OD686** | **Cell number** | **Concentration** | **Amount of DNA** | **Vortexing** | **Results** |
|---|---|---|---|---|---|---|
| 160810 | 0.1 | 1*10⁶cells/ml | 1*10⁸cells/ml | 10 ug linearized | 15 sec | NO |
| 160811 | 0.3 | 1*10⁶cells/ml | 1*10⁸cells/ml | 10 ug linearized | 10 sec | NO |
| 160816 | 0.3 | 1*10⁷cells/ml | 1*10⁹cells/ml | 10 ug non-linearized | 20 sec | NO |

As could be confirmed in FIG. 2, it was confirmed that the efficiency was low and the transformation did not proceed successfully.

### 2-2. Transformation using a gene gun method

Transformation was conducted using gold particles bombardment using a gene gun, which is a plant transformation method. This technique is also called microparticle acceleration or biolistics, but the official name of the device called a gene gun is microparticle bombardment. It is a method of transformation by coating plasmids with microparticles. Since microparticles are very heavy relative to their size, they can penetrate cells well. Microparticles are sent at high speed towards the cells and the surroundings were covered with steel mesh, allowing the particles to fly into the cells more. DNA coated on microparticles entering the cells are released and can enter the plant' s genome. Transformation was performed using gold with these microparticles.

Specifically, as Table 2 below, to perform transformation by gold particles bombardment, experiments were conducted by varying several conditions. First, Vacuum and Helium Pressure were fixed, and the experiments were performed considering the cell stage and concentration, dispersion range of gold particles, target distance, and experimental conditions of cells.

In the process of establishing the conditions, considering the dispersion range of gold particles and pressure of the gene gun, the experiments were conducted at 47mm, 50mm, 60mm, and the experiments were performed with Target Distance 3 as the appropriate position by proceeding the distance of Target Distance with changes. In addition, when the cell stage progresses significantly, the membrane of microalgae becomes thicker, making the experiments difficult, so the experiments were conducted according to changes in OD values from the early stage. Furthermore, the experiments were conducted by adjusting the cell density based on the diameter.

**[Table 2]**

| **Date** | **OD686** | **Cell Density** | **Vacuum (inchs Hg)** | **Target Distance** | **Helium Pressure** | **Results** |
|---|---|---|---|---|---|---|
| 160603 | 0.5 | 4.0*107 per 60mm Diameter | 29 | 3,6,9 | 1300 | NO |
| 160607 | 0.7 | 6.0*107 per 50mm Diameter | 29 | 3 | 1300 | NO |
| 160607 | 0.7 | 6.0*108 per 50mm Diameter | 29 | 6 | 1300 | NO |
| 160607 | 0.7 | 6.0*109 per 50mm Diameter | 29 | 9 | 1300 | NO |
| 160613 | 0.074 | 3.6*106 per 50mm Diameter | 29 | 3,6,9 | 1300 | NO |
| 160614 | 0.1 | 3.6*106 per 50mm Diameter | 29 | 3.6.9 | 1300 | NO |
| 160615 | 0.2 | 3.5*106 per 50mm Diameter | 29 | 3.6.9 | 1300 | NO |
| 160616 | 0.3 | 4.0*106 per 50mm Diameter | 29 | 3.6.9 | 1300 | NO |
| 160617 | 0.6 | 3.6*106 per 50mm Diameter | 29 | 3.6.9 | 1300 | NO |
| 160629 | 0.1 | 4.8*107 per 60mm Diameter | 29 | 3.6.9 | 1300 | NO |
| 160630 | 0.2 | 4.8*107 per 60mm Diameter | 29 | 3.6.9 | 1300 | NO |
| 160701 | 0.3 | 4.8*107 per 60mm Diameter | 29 | 3.6.9 | 1300 | NO |
| 160704 | 0.6 | 4.8*107 per 60mm Diameter | 29 | 3.6.9 | 1300 | YES |
| 160711 | 0.1 | 1.0*107 per 47mm Membrane | 29 | 3 | 1300 | NO |
| 160712 | 0.2 | 1.0*107 per 47mm Membrane | 29 | 3 | 1300 | NO |
| 160713 | 0.3 | 1.0*107 per 47mm Membrane | 29 | 3 | 1300 | NO |
| 160714 | 0.6 | 1.0*107 per 47mm Membrane | 29 | 3 | 1300 | NO |
| 160815 | 0.07 | 2.0*107 per 47mm Membrane | 29 | 3 | 1300 | - |
| 160816 | 0.1 | 2.0*107 per 47mm Membrane | 29 | 3 | 1300 | - |
| 160818 | 0.3 | 2.0*107 per 47mm Membrane | 29 | 3 | 1300 | - |

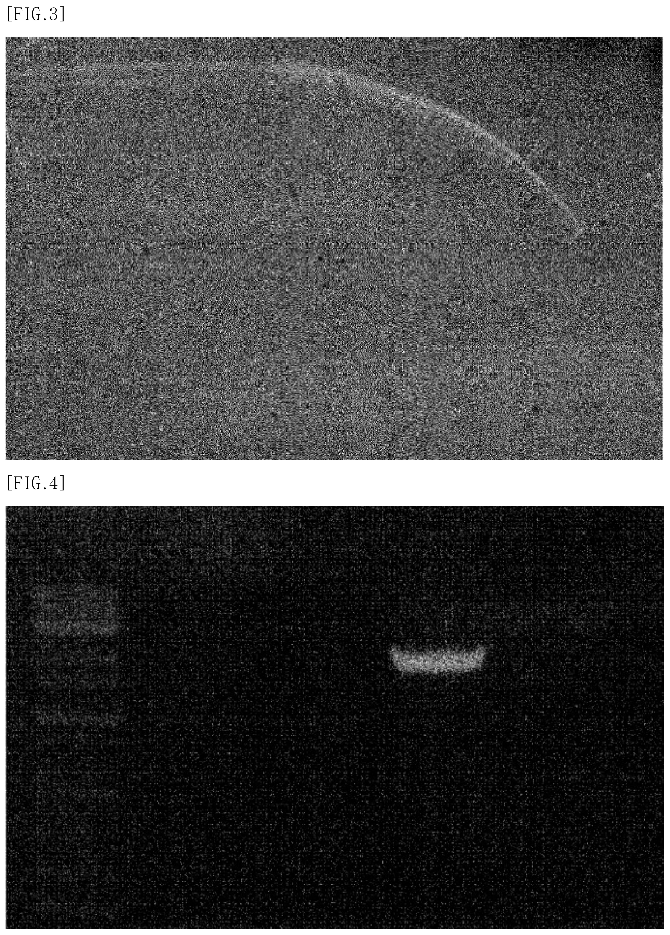

As could be confirmed in Table 2 and FIG. 3, in case of failure, cells do not grow on solid media containing an antibiotic, and in case of success, colonies were formed, so only in case of the conditions of 160704, operation (mutants) of the promoter was confirmed. Then, mutants were generated by introducing the target protein (Carbonic anhydrase) under the same conditions.

### Experimental example 1. Confirmation of genetic information

In order to confirmation of genetic information thereof, genomic DNA was secured and the introduced gene was confirmed by PCR and DNA sequencing. Specifically, in order to perform PCR for identifying a gene, the primer sets of Table 3 below were produced, and with the primer sets, pre denaturation was performed at 98 degrees for 8 minutes, and PCR was performed at 72 degrees for 7 minutes for 30 cycles of 98 degrees for 1 minute, 53.5 degrees for 30 seconds, and 72 degrees for 1 minute, and this was confirmed by sequencing analysis. This was shown in Table 1.

In addition, in order to confirm *Sh-ble,* which is the entire sequence obtained by conjugating the promoter sequence of the *Coccomyxa* C-169 *rbcS2* gene at the 5' end, and the terminator sequence of the *Coccomyxa* C-169 *rbcS2* gene at the 3' end of the target bleomycin resistance gene of the present inventors, PCR was performed with M13 primer set. Pre denaturation was performed at 98 degrees for 8 minutes, and PCR was performed at 72 degrees for 7 minutes for 30 cycles of 98 degrees for 1 minute, 54 degrees for 40 seconds, and 72 degrees for 2 minutes and 30 seconds, and the target DNA band was confirmed through PCR, and the results were shown in FIG. 4.

**[Table 3]**

| SEQ ID NO: | Name | Sequence listing (5'-> 3') | Note |
|---|---|---|---|
| 5 | Forward primer | ATGGCCAAGTTGACCAGT | |
| 6 | Reverse primer | TCAGTCCTGCTCCTCGGCCA | |

As could be confirmed in FIG. 4, a band was identified at about 2kb, which is the total size of the target gene. By confirming whether this matches the target gene through DNA gene analysis, the results were shown in Table 4.

**[Table 4]**

| | |
|---|---|
| sh-ble TF5ble1 | ------------------------------------------ ------------------ CGGCAATGACTGATTACGCCAGCTTGGTACCGAGCTCGGATC CACTAGTAACGGCCGCCA |
| sh-ble TF5ble1 | -------------------- ATGGCCAAGTTGACCAGTGCCGTTCCGGTGCTCACCGCGC GTGTGCTGGAATTCGCCCTTATGGCCAAGTTGACCAGTGCCG TTCCGGTGCTCACCGCGC **************************************** |
| sh-ble | GCGACGTCGCCGGAGCGGTCGAGTTCTGGACCGACCGGCTCG |
| TF5ble1 | GGTTCTCCCGGGACTTCG GCGACGTCGCCGGAGCGGTCGAGTTCTGGACCGACCGGCTCG GGTTCTCCCGGGACTTCG ****************************************** ****************** |
| sh-ble TF5ble1 | TGGAGGACGACTTCGCCGGTGTGGTCCGGGACGACGTGACCC TGTTCATCAGCGCGGTCC TGGAGGACGACTTCGCCGGTGTGGTCCGGGACGACGTGACCC TGTTCATCAGCGCGGTCC ****************************************** ****************** |
| sh-ble TF5ble1 | AGGACCAGGTGGTGCCGGACAACACCCTGGCCTGGGTGTGGG TGCGCGGCCTGGACGAGC AGGACCAGGTGGTGCCGGACAACACCCTGGCCTGGGTGTGGG TGCGCGGCCTGGACGAGC ****************************************** ****************** |
| sh-ble TF5ble1 | TGTACGCCGAGTGGTCGGAGGTCGTGTCCACGAACTTCCGGG ACGCCTCCGGGCCGGCCA TGTACGCCGAGTGGTCGGAGGTCGTGTCCACGAACTTCCGGG ACGCCTCCGGGCCGGCCA ****************************************** ****************** |
| sh-ble TF5ble1 | TGACCGAGATCGGCGAGCAGCCGTGGGGGCGGGAGTTCGCCC TGCGCGACCCGGCCGGCA TGACCGAGATCGGCGAGCAGCCGTGGGGGCGGGAGTTCGCCC |
| | TGCGCGACCCGGCCGGCA ****************************************** ****************** |
| sh-ble TF5ble1 | ACTGCGTGCACTTCGTGGCCGAGGAGCAGGACTGA ACTGCGTGCACTTCGTGGCCGAGGAGCAGGACTGA *********************************** |

As could be confirmed in Table 4, as a result of CLUSTAL 2.1 multiple sequence alignment, it was confirmed that the DNA base sequence of the target gene, Sh-ble, was completely identical, when compared with the secured genetic information.

### Experimental example 2. Confirmation of maintenance of mutants

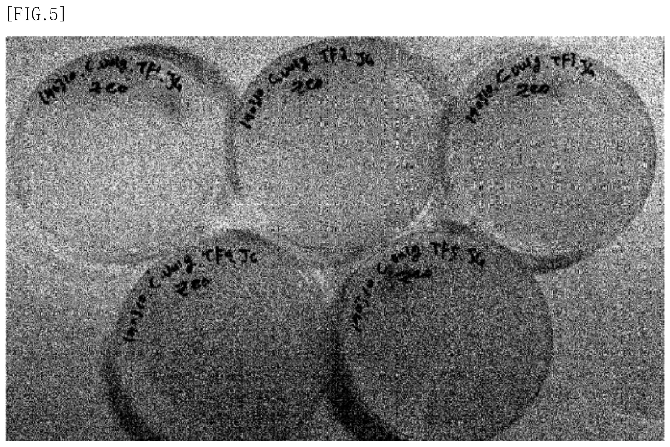

For mutant maintenance, mutant maintenance was confirmed by subculturing, and the result was shown in FIG. 5. As could be confirmed in FIG. 5, for mutant maintenance, mutant maintenance was confirmed by subculturing.

### Experimental example 3. Biomineralization

### Example 1: Plasmid construct and cloning

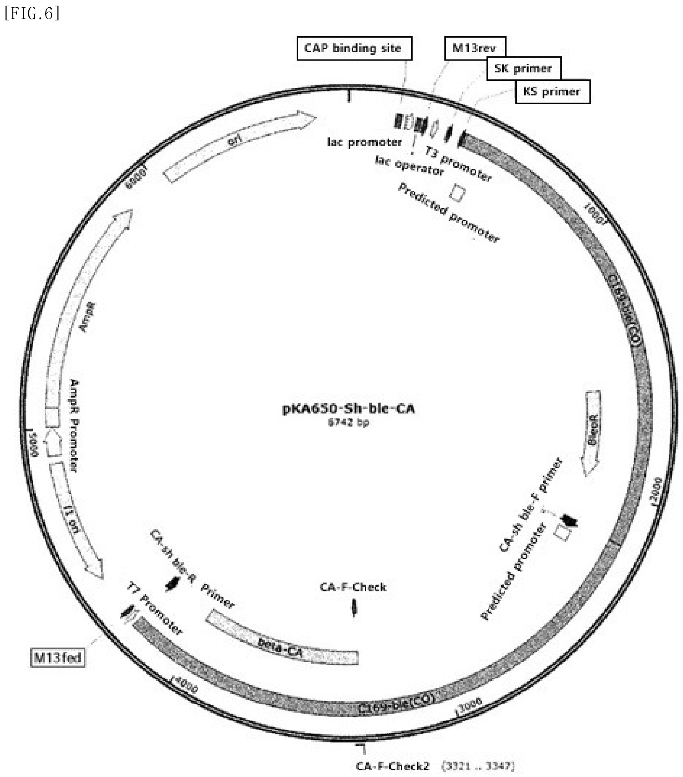

This is construction and cloning of a plasmid having resistance to zeocin for expressing the target protein by inserting a gene for a target protein based on the pKA650 vector. The sequence of the bleomycin resistance gene (*Sh ble*) was obtained from genomic DNA of *Streptomyces verticillus* (*Sh ble*). The entire sequence generated by attaching the promoter and terminator sequences of the *Coccomyxa* C-169 rbcS2 (ribulose-1,5-bisphosphate carboxylase/oxygenase small subunit) gene at the 5' and 3' regions of *Sh ble,* respectively, was synthesized. This sequence was named pKA650, and from the synthesized pKA650, the intermediate gene, *BleoR,* of the full sequence, *Sh-ble,* was synthesized by substituting the C-169 *beta-type carbonic anhydrase* gene, and then duplicate promoter regions were inserted using restriction enzymes, *Kpn* I, *Apa* I*.* The cloned plasmid was shown in FIG. 6, and this was named pJG002.

### Example 2: Transformation of Chlorella vulgaris

The transformation method by the pJG002 vector is the same as the transformation method by the pKA650 vector.

Specifically, in order to prepare competent cells, 300mℓ sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L) was prepared in a 500mL flask, and *Chlorella vulgaris* was inoculated and cultured under the conditions of 23°C and 100 rpm. When the OD₆₈₆ value reached 0.5-0.6, *Chlorella vulgaris* was harvested and then the number of the cells was confirmed.

After confirming the number of the cells, they were prepared with the cell density of 4.8*10⁷. After that, for gold particles bombardment, pKA650 to be introduced was prepared as linearized with the restriction enzyme KpnI. Retaining cap, Brass Adjustable Nest, Micro carrier Holder, Stopping screen, and Macrocarrier, which were prepared as pre-sterilized, were prepared, and the rupture disk was washed with 70% isopropanol. While the rupture disk was dried, the plasmid and gold particle mixture were prepared. First, for 20 bombardments, 12mg of gold was placed into a microfuge tube, and 1ml of 90% ethanol was added. Vortexing was conducted at maximum speed for 5 minutes, and a centrifuge was operated to remove the supernatant.

The gold particles were washed by repeating the order of 1ml of distilled water, vertexing for 1 minute, letting them stand for 1 minute, and operating the centrifuge for 5 seconds, and then removing the supernatant, three times. Then, 205ul of 50% glycerol was added, and it was released by vortexing for 5 minutes. Vortexing was continued at a vortexing speed of 2~3, and for 10 bombardments, 100ul of gold particles were moved to a new microfuge tube, and then while vortexing, 10ul DNA (0.5-20ug/ul), 100ul 2.5M CaCl₂, and 40ul 0.1M spermidine were added in order and they were mixed by pipetting. After mixing, vortexing was continued for 2 minutes and it was left standing for 1 minute. It was spun down with the centrifuge for 2 seconds, and the supernatant was removed. When the supernatant was removed, 300ul of 70% ethanol was added and it was left for 1 minute and the supernatant was removed, and then 300ul of 100% ethanol was added and it was left for 1 minute. It was removed again, and 110ul of 100% ethanol was added and pellets were released by continuous vortexing.

When the Gold Particle Mix was completed, 11ul was placed in the center of a microcarrier and dried for 5-10 minutes. Meanwhile, the prepared cells were evenly spread on the medium in a 60mm diameter. Then, a stopping screen was placed on the Brass Adjustable Nest and the completely dried microcarrier was placed. Next, it was fixed with a holder, and a gene gun instrument was turned on and preheated for 5 minutes. When preheating was completed, the rupture disk was inserted into the retaining cap and screwed onto the gun part. Then, the Brass Adjustable Nest combined with the microcarrier was inserted, and a medium in which cells were spread at Target distance 3 was placed and the door was closed. The helium gas was opened and the vacuum was pressed. When the vacuum reached 29 (inchs Hg), Helium Pressure 1300psi, the fire button was pressed and held, and the pressure increased to 1100psi, the gold particles were shot. After bombardment, the medium had a recovery phase by incubation at 23 degrees.

### Example 3: Confirmation of Chlorella vulgaris transformants

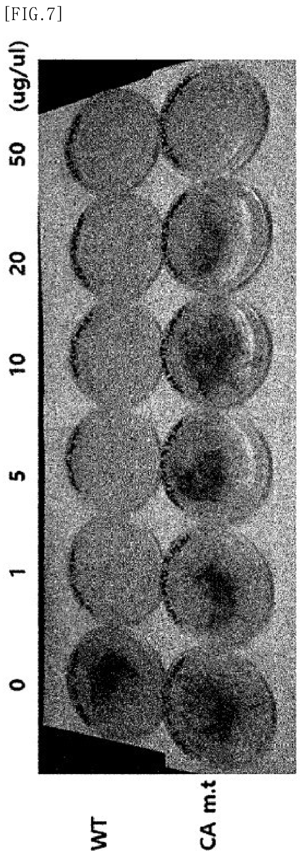

Transformation was conducted biolistically as in Example 2, and the maintenance of antibiotic resistance after subculturing was confirmed by concentration, and shown in FIG. 7. As a total transformants, 42 transformants were obtained. For them, subculturing was continued during irradiating light using the medium in Example 2.

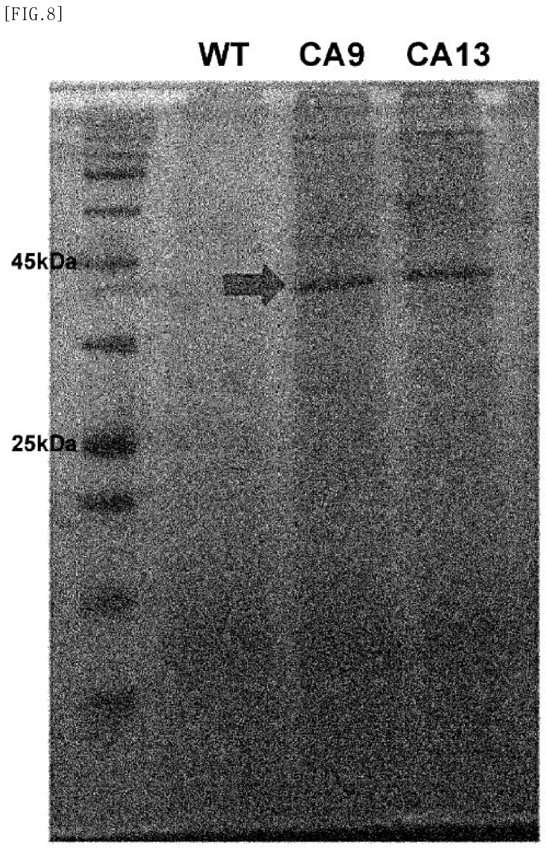

In order to confirm gene, protein expression of these transformants, the band size of about 43kDa of the target protein, beta-type carbonic anhydrase, was confirmed by whole protein SDS-PAGE, and the result was shown in FIG. 8.

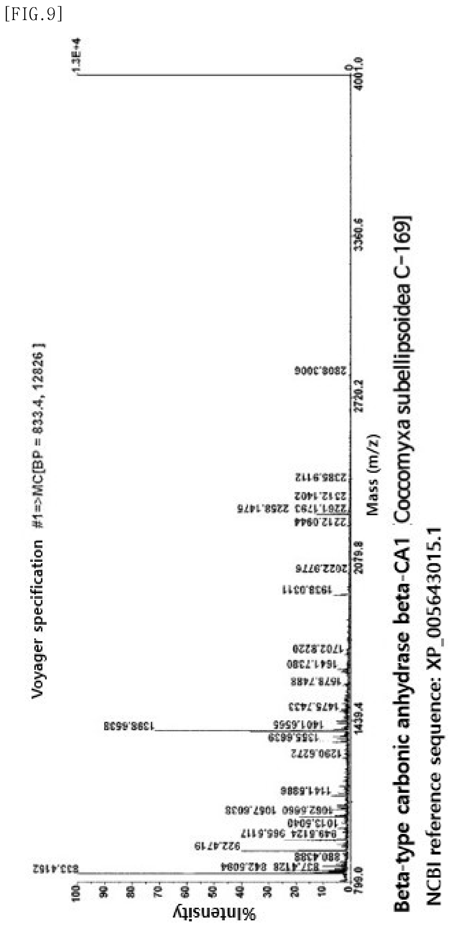

To confirm the band of this target protein, MALDI-TOF was conducted, and the result was shown in FIG. 9.

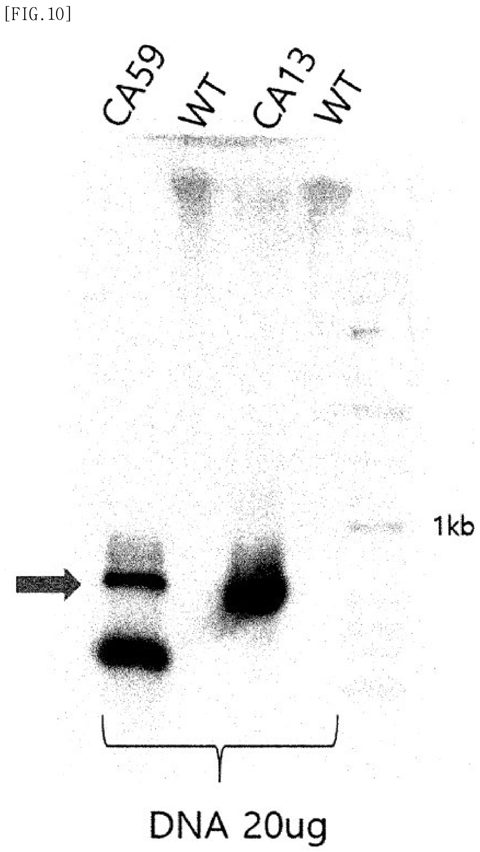

The present invention confirmed protein expression of the transformants, and confirmed the maintenance of subculturing for resistance, thereby progressing the invention, and for genetic confirmation, the presence or absence of the target gene was confirmed through southern blot, and the result was shown in FIG. 10.

As could be confirmed in FIG. 10, the band of DNA was identified at the same position as the size of the target gene, and this was also confirmed by DNA sequencing through PCR.

### Example 4: Sr biomineralization

The function of the transformants was confirmed by visualizing crystallization in the same amount for biomineralized crystals through a microscope to provide enhancement of the function for Sr biomineralization. First, the Sr biomineralization ability of the wild type (WT), *Chlorella vulgaris,* was confirmed.

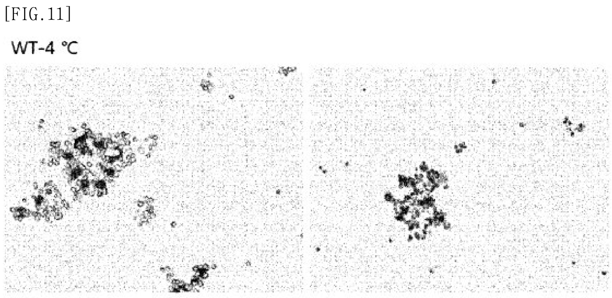

Specifically, *Chlorella vulgaris* was cultured under light conditions until OD 0.6. After culturing, microalgae were collected under the condition of maintaining sterilization under the conditions of 25 degrees, 4000rpm, 15 minutes. Then, the microalgae were washed with 3mM NaHCO3 three times. After that, cells were adjusted to 1*10^7 and Sr 200ppm was added to the prepared 3mM NaHCO3, and then, the prepared *Chlorella vulgaris* was mixed and cultured at 4 degrees for 4 hours or more. Then, it was stained with sodium rhodizonate 0.004% and observed through a microscope, and the result was shown in FIG. 11.

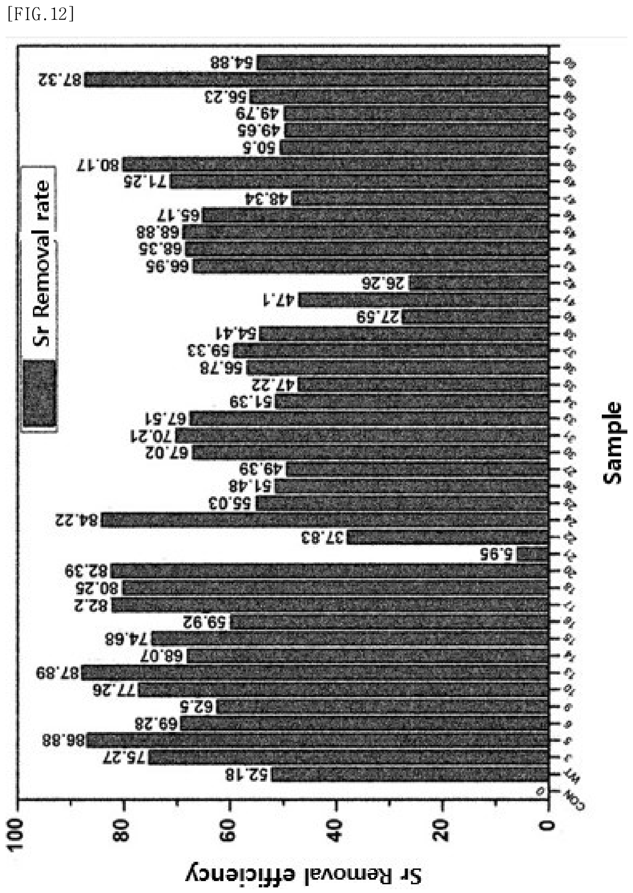

As could be confirmed in FIG. 11, the crystallization of WT confirmed a low amount of biomineralized crystals compared to the transformants. In addition, for quantitative comparison, analysis was performed through ICP-MS, and the result was shown in FIG. 12 and Table 5.

**[Table 5]**

| | Remain metal ion rate (%) | RSD(%) : Relative |
|---|---|---|
| | | Standard Deviation |
| Control (200 mM LiCl) | 100 | 0.56 |
| + algae | 45.27667 | 0.42 |

As could be confirmed in FIG. 12 and Table 5, it was confirmed that the transformants had biomineralized crystals increased by about 160% at maximum than the wild type.

### Example 5. Results of Lithium (Li) biomineralization in single LiCl

### 5-1. Removal rate according to LiCl Concentration

Using the transformed *Chlorella vulgaris* (cell) in Example 3, LiCl (sigma), sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L), DIW (deionized water), white light, a stirrer, a 10ml Cornical tube, a centrifuge, and a 0.2uM syringe filter, the removal rate of lithium crystals according to the lithium concentration of a single LiCl solution was confirmed. The specific experimental method was as follows.

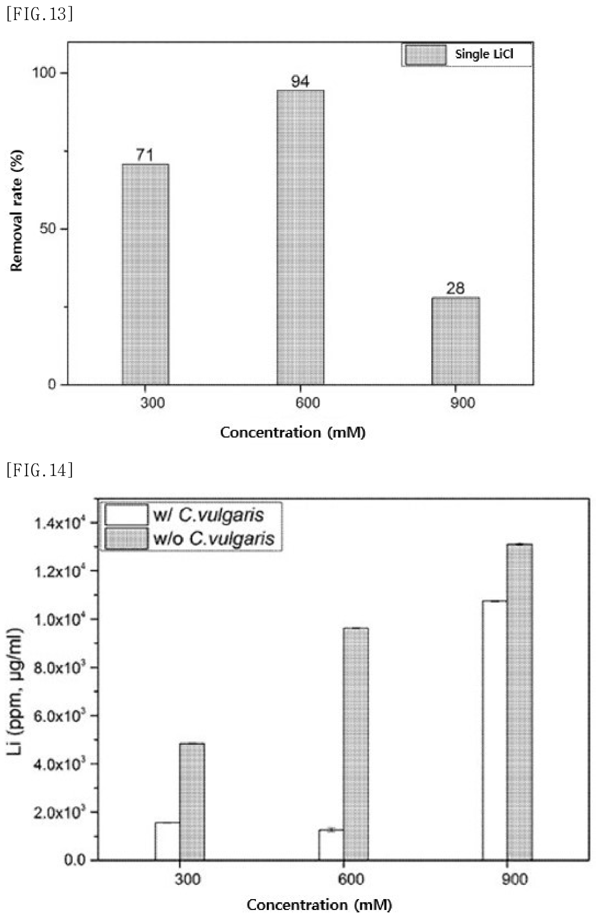

Fresh (log phase) cells of *Chlorella vulgaris* were cultured in the MBM medium by 6*10^7/ml. After harvesting 5ml of the cells collected in the medium, they were mixed with 5ml of the LiCl solution by concentration (each 300, 600 and 900 mM) using a 10ml Cornical tube to mix them by 10ml in total. This was slowly stirred and reacted under white light for 16 hours. After 16 hours, at 3000rpm, centrifugation was performed for 3 minutes. The cells and crystals were filtered by a 0.2uM syringe filter by collecting the supernatant, and the collected solution was used for ICP analysis. Samples were ionized using inductively coupled plasma measured by loading the samples into ICP-OES and MS instruments, and then the corresponding ions were separated using a mass spectrometer and ICP analysis was performed. The result was shown in FIG. 13 to FIG. 14.

Through the result of FIG. 13, the lithium removal rate according to the concentration of lithium was confirmed. It was confirmed that the lithium removal rates of about 71% at 300 mM, about 94% at 600 mM, and about 28% at 900 mM were shown.

In addition, through the result of FIG. 14, the lithium removal rate according to the presence or absence of cells was confirmed. It was confirmed that a significantly excellent lithium removal rate was shown in case of presence of the cells (w/ *C.vulgaris*) compared to the case when the cells were not present (w/o *C.vulgaris*) at all concentrations, and the specific measurement values of FIG. 14 were shown in Table 6 below.

**[Table 6]**

| | 300 mM | | 600 mM | | 900 mM | |
|---|---|---|---|---|---|---|
| | w/ *C. vulgaris* | w/o *C. vulgaris* | w/ *C. vulgaris* | w/o *C. vulgaris* | w/ *C. vulgaris* | w/o *C. vulgaris* |
| Li (ppm, µg/ml) | 1561.8039 | 4847.02505 | 1260.30268 | 9625.45826 | 10744. 2233 | 13102.87375 |

### 5-2. Effect results on Sodium and Sulfate

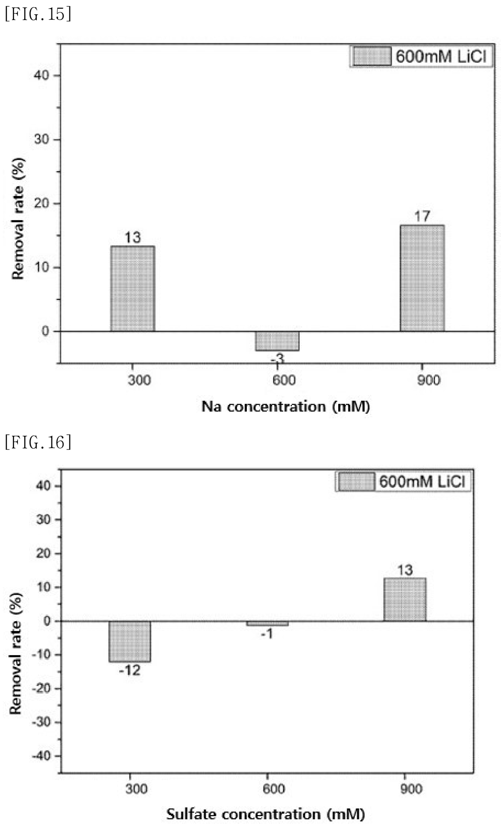

In order to measure the effect results on sodium and sulfate, experiments were performed by the same method as Example 5-1, but NaCl or potassium sulfate was further added to the Cornial tube by concentration (300, 600, 900mM). The concentration of Li was fixed at 600mM. The results were shown in FIG. 15 to FIG. 16.

As a result, through the result of FIG. 15, the lithium removal rate according to the concentration of sodium was confirmed. It was confirmed that the lithium removal rates of about 13% at 300 mM, about -3% at 600 mM, and about 17% at 900 mM were shown. In other words, it was confirmed that the constant correlation between the concentration of sodium and lithium removal rate was not shown.

In addition, through the result of FIG. 16, the lithium removal rate according to the concentration of sulfate was confirmed. It was confirmed that the lithium removal rates of about -12% at 300 mM, about -1% at 600 mM and about 13% at 900 mM were shown. In other words, it was confirmed that the constant correlation between the concentration of sulfate and lithium removal rate was not shown.

### Example 6. Preparation of lithium battery solution

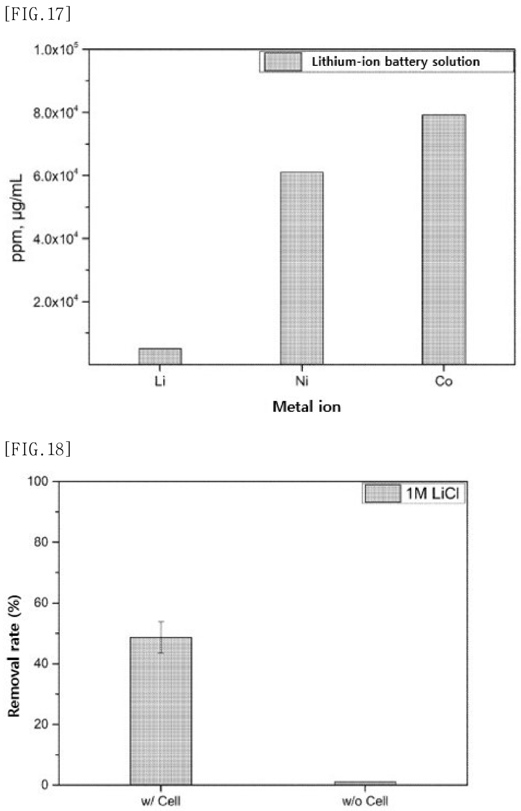

A lithium battery solution was prepared by mixing LiCl (sigma), NiCl₂ (sigma), CoCl₂ (sigma), MnCl (sigma), AlCl₃ (sigma), CuCl₂ (sigma), FeCl₂ (sigma), ClF (sigma), KH₂PO₄ (sigma) to DIW (deionized water). The composition of the prepared lithium battery solution was analyzed and shown in Table 7 and FIG. 17 below.

**[Table 7]**

| **Elements** | **g per 1L** |
|---|---|
| Al | 5 |
| Co | 33 |
| Cu | 3 |
| Fe | 0.3 |
| Li | 4 |
| Mn | 11 |
| Ni | 26 |
| F | 4 |
| P | 1 |

As could be confirmed in Table 7, it was confirmed that it was Li 4% (1M), Ni 26% (1.09M), and Co 33% (1.39M).

### Example 7. Results of Lithium (Li) biomineralization in lithium battery solution

### 7-1. Crystallization in lithium battery solution

The Li crystallization result was confirmed in the lithium battery solution, using the transformed *Chlorella vulgaris* (cell) in Example 3, the lithium battery solution prepared in Example 6, sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L), DIW (deionized water), white light, a stirrer, a 10ml Cornical tube, a centrifuge, and a 0.2uM syringe filter. The specific experimental method was as follows.

Fresh (log phase) cells of *Chlorella vulgaris* were cultured in the MBM medium by 6*10^7/ml. After harvesting 5ml of the cells collected in the medium, they were mixed with lithium battery solution containing 5ml of 2M LiCl using a 10ml Cornical tube, and finally, 10ml of 1M LiCl solution was prepared. This was slowly stirred and reacted under white light for 16 hours. After 16 hours, at 3000rpm, centrifugation was performed for 3 minutes. The cells and crystals were filtered by a 0.2uM syringe filter by collecting the supernatant, and the collected solution was used for ICP analysis. Samples were ionized using inductively coupled plasma measured by loading the samples into ICP-OES and MS instruments, and then the corresponding ions were separated using a mass spectrometer and ICP analysis was performed. The result was shown in FIG. 18.

Through the result of FIG. 18, the lithium removal rate according to the presence or absence of cells was confirmed. It was confirmed that lithium was hardly removed when cells were not present (w/o Cell), but lithium of about 50% was removed when the cells were present (w/ Cell).

### 7-2. Crystallization according to Na, S in lithium battery solution

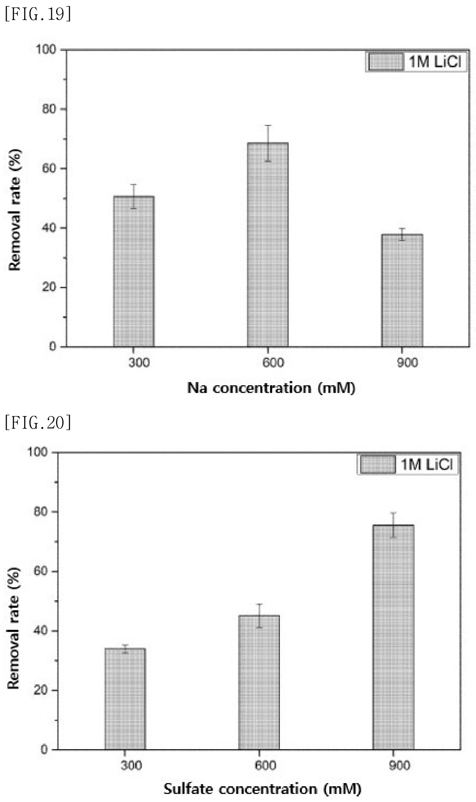

In order to measure the effect results on sodium and sulfate, experiments were performed by the same method as Example 7-1, but NaCl or potassium sulfate was further added to the Cornial tube by concentration (300, 600, 900mM). The results were shown in FIG. 19 to FIG. 20.

Through the result of FIG. 19, the lithium removal rate according to the concentration of sodium was confirmed. It was confirmed that the lithium removal rates of about 56% at 300 mM, about 68% at 600 mM, and about 37% at 900 mM were shown. In other words, it was confirmed that the constant correlation between the concentration of sodium and lithium removal rate was not shown.

In addition, through the result of FIG. 20, the lithium removal rate according to the concentration of sulfate was confirmed. It was confirmed that the lithium removal rates of about 33% at 300 mM, about 45% at 600 mM and about 75% at 900 mM were shown. In other words, it was confirmed that the lithium removal rate increased as the concentration of sulfate increased.

### Example 8. Results of Nickel (Ni) biomineralization in single NiCl2

### 8-1. Removal rate according to NiCl2 Concentration

The removal rate according to the nickel concentration in a single NiCl₂ solution was confirmed, using the transformed *Chlorella vulgaris* (cell) in Example 3, NiCl₂ (sigma), sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L), DIW (deionized water), white light, a stirrer, a 10ml Cornical tube, a centrifuge, and a 0.2uM syringe filter. The specific experimental method was as follows.

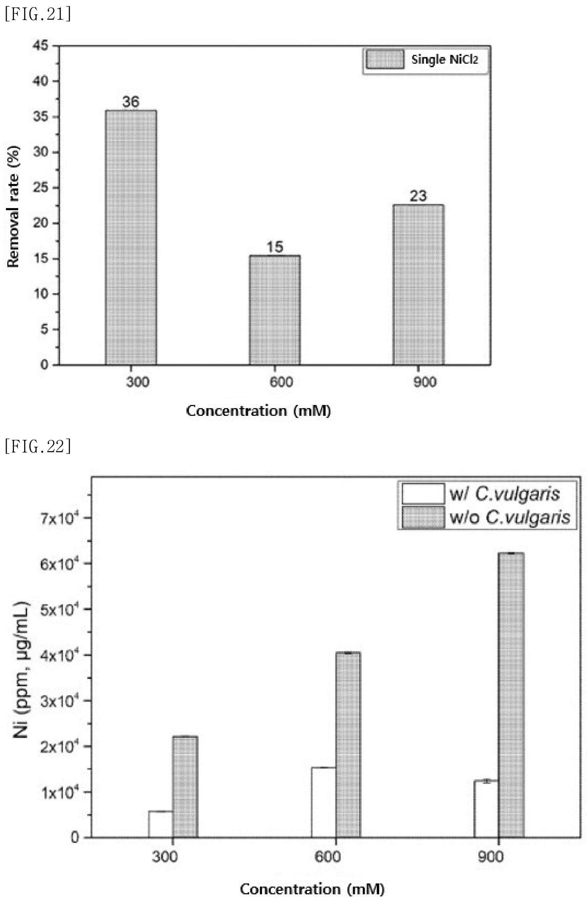

Fresh (log phase) cells of *Chlorella vulgaris* were cultured in the MBM medium by 6*10^7/ml. After harvesting 5ml of the cells collected in the medium, they were mixed with 5ml of NiCl₂ solution by concentration (each 300, 600 and 900 mM) using a 10ml Cornical tube, to mix them by 10ml in total. This was slowly stirred and reacted under white light for 16 hours. After 16 hours, at 3000rpm, centrifugation was performed for 3 minutes. The cells and crystals were filtered by a 0.2uM syringe filter by collecting the supernatant, and the collected solution was used for ICP analysis. Samples were ionized using inductively coupled plasma measured by loading the samples into ICP-OES and MS instruments, and then the corresponding ions were separated using a mass spectrometer and ICP analysis was performed. The results were shown in FIG. 21 to FIG. 22.

Through the result of FIG. 21, the nickel removal rate according to the concentration of nickel was confirmed. It was confirmed that the nickel removal rates of about 36% at 300 mM, about 15% at 600 mM, and about 23% at 900 mM were shown.

In addition, through the result of FIG. 22, the nickel removal rate according to the presence or absence of cells was confirmed. It was confirmed that a significantly excellent nickel removal rate was shown in case of presence of the cells (w/ *C.vulgaris*) compared to the case when the cells were not present (w/o *C.vulgaris*) at all concentrations, and the specific measurement values of FIG. 14 were shown in Table 8 below.

**[Table 8]**

| | 300 mM | | 600 mM | | 900 mM | |
|---|---|---|---|---|---|---|
| | w/ *C. vulgaris* | w/o *C. vulgaris* | w/ *C. vulgaris* | w/o *C. vulgaris* | w/ *C. vulgaris* | w/o *C. vulgaris* |
| Ni (ppm, µg/ml) | 5767.98 | 22184.54 | 15376.39 | 40464.19 | 12454.16 | 62270.82 |

### 8-2. Effect results on Sodium and Sulfate

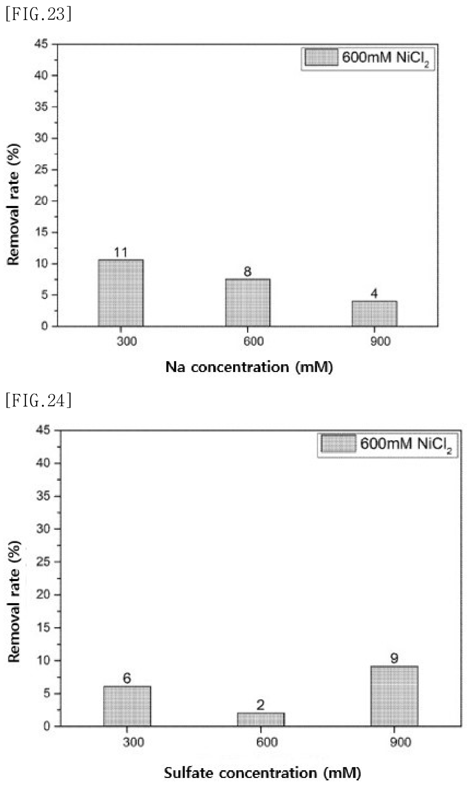

In order to measure the effect results on sodium and sulfate, experiments were performed by the same method as Example 8-1, but NaCl or potassium sulfate was further added to the Cornial tube by concentration (300, 600, 900mM). The concentration of Ni was fixed at 600mM. The results were shown in FIG. 23 to FIG. 24.

As a result, through the result of FIG. 23, the nickel removal rate according to the concentration of sodium was confirmed. It was confirmed that the nickel removal rates of about 11% at 300 mM, about 8% at 600 mM, and about 4% at 900 mM were shown. In other words, it was confirmed that the nickel removal rate was reduced as the concentration of sodium increased.

In addition, through the result of FIG. 24, the nickel removal rate according to the concentration of sulfate was confirmed. It was confirmed that the nickel removal rates of about 6% at 300 mM, about 2% at 600 mM and about 9% at 900 mM were shown. In other words, it was confirmed that the constant correlation between the concentration of sulfate and nickel removal rate was not shown.

### Example 9. Preparation of nickel battery solution

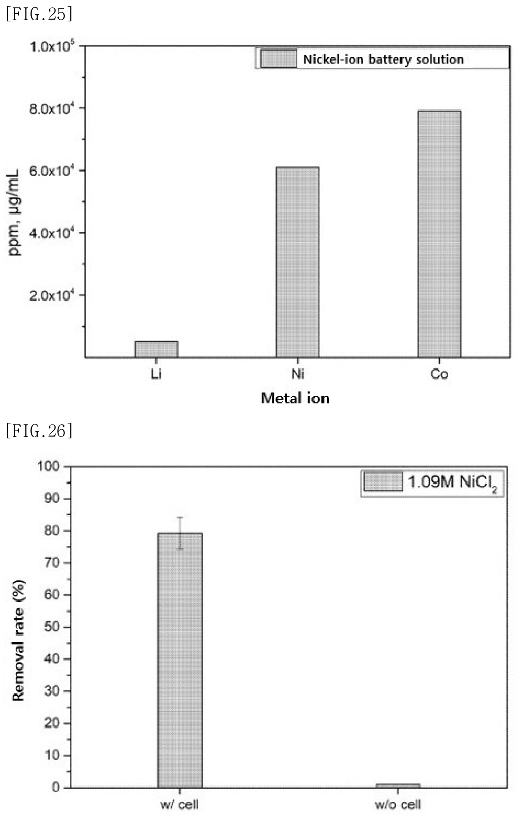

A nickel battery solution was prepared by mixing LiCl₂ (sigma), NiCl₂ (sigma), CoCl₂ (sigma), MnCl (sigma), AlCl₃ (sigma), CuCl₂ (sigma), FeCl₂ (sigma), ClF (sigma), KH₂PO₄ (sigma) to DIW (deionized water). The composition of the prepared nickel battery solution was analyzed and shown in Table 9 and FIG. 25 below.

**[Table 9]**

| **Elements** | **g per 1L** |
|---|---|
| Al | 5 |
| Co | 33 |
| Cu | 3 |
| Fe | 0.3 |
| Li | 4 |
| Mn | 11 |
| Ni | 26 |
| F | 4 |
| P | 1 |

As could be confirmed in Table 9, it was confirmed that it was Li 4%(1M), Ni 26%(1.09M), and Co 33%(1.39M).

### Example 10. Results of Nickel (Ni) biomineralization in nickel battery solution

### 10-1. Crystallization in nickel battery solution

The Ni crystallization result was confirmed in the nickel battery solution, using the transformed *Chlorella vulgaris* (cell) in Example 3, the nickel battery solution prepared in Example 9, sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L), DIW (deionized water), white light, a stirrer, a 10ml Cornical tube, a centrifuge, and a 0.2uM syringe filter. The specific experimental method was as follows.

Fresh (log phase) cells of *Chlorella vulgaris* were cultured in the MBM medium by 6*10^7/ml. After harvesting 5ml of the cells collected in the medium, they were mixed with nickel battery solution containing 5ml of 2.18M NiCl₂ using a 10ml Cornical tube, and finally, 10ml of 1.09M NiCl₂ solution was prepared. This was slowly stirred and reacted under white light for 16 hours. After 16 hours, at 3000rpm, centrifugation was performed for 3 minutes. The cells and crystals were filtered by a 0.2uM syringe filter by collecting the supernatant, and the collected solution was used for ICP analysis. Samples were ionized using inductively coupled plasma measured by loading the samples into ICP-OES and MS instruments, and then the corresponding ions were separated using a mass spectrometer and ICP analysis was performed. The result was shown in FIG. 26.

Through the result of FIG. 26, the nickel removal rate according to the presence or absence of cells was confirmed. It was confirmed that nickel was hardly removed when cells were not present (w/o Cell), but nickel of about 80% was removed when the cells were present (w/ Cell).

### 10-2. Crystallization according to Na, S in nickel battery solution

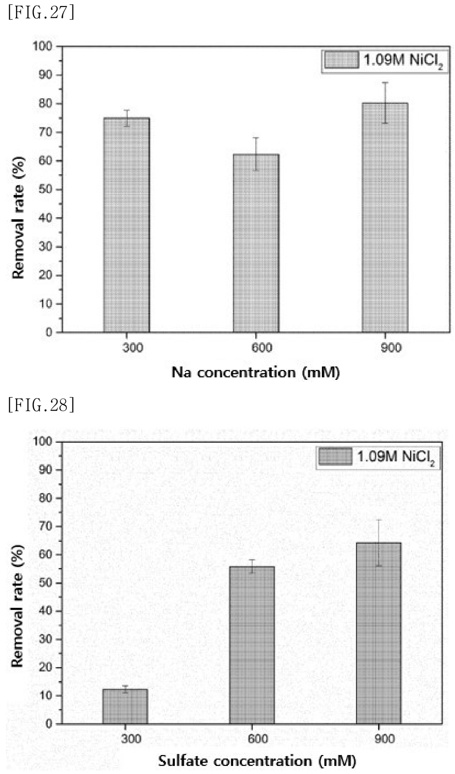

In order to measure the effect results on sodium and sulfate, experiments were performed by the same method as Example 10-1, but NaCl or potassium sulfate was further added to the Cornial tube by concentration (300, 600, 900mM). The results were shown in FIG. 27 to FIG. 28.

Through the result of FIG. 27, the nickel removal rate according to the concentration of sodium was confirmed. It was confirmed that the nickel removal rates of about 74% at 300 mM, about 62% at 600 mM, and about 80% at 900 mM were shown. In other words, it was confirmed that the constant correlation between the concentration of sodium and nickel removal rate was not shown.

In addition, through the result of FIG. 28, the nickel removal rate according to the concentration of sulfate was confirmed. It was confirmed that the nickel removal rates of about 12% at 300 mM, about 55% at 600 mM and about 64% at 900 mM were shown. In other words, it was confirmed that the nickel removal rate increased as the concentration of sulfate increased.

### Example 11. Results of Cobalt (Co) biomineralization in single CoCl2

### 11-1. Removal rate according to CoCl2 concentration

The removal rate according to the cobalt concentration of a single CoCl₂ solution was confirmed, using the transformed *Chlorella vulgaris* (cell) in Example 3, CoCl₂ (sigma), sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L), DIW (deionized water), white light, a stirrer, a 10ml Cornical tube, a centrifuge, and a 0.2uM syringe filter. The specific experimental method was as follows.

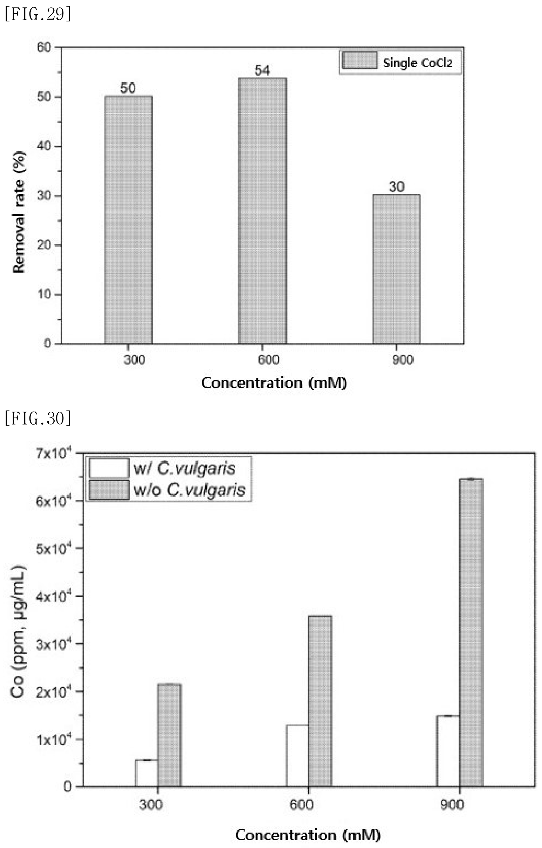

Fresh (log phase) cells of *Chlorella vulgaris* were cultured in the MBM medium by 6*10^7/ml. After harvesting 5ml of the cells collected in the medium, they were mixed with 5ml of CoCl₂ solution by concentration (each 300, 600 and 900 mM) using a 10ml Cornical tube, and finally, a total of 10ml was prepared. This was slowly stirred and reacted under white light for 16 hours. After 16 hours, at 3000rpm, centrifugation was performed for 3 minutes. The cells and crystals were filtered by a 0.2uM syringe filter by collecting the supernatant, and the collected solution was used for ICP analysis. Samples were ionized using inductively coupled plasma measured by loading the samples into ICP-OES and MS instruments, and then the corresponding ions were separated using a mass spectrometer and ICP analysis was performed. The result was shown in FIG. 29 to FIG. 30.

Through the result of FIG. 29, the cobalt removal rate according to the concentration was confirmed. It was confirmed that the cobalt removal rates of about 50% at 300 mM, about 54% at 600 mM and about 30% at 900 mM were shown.

In addition, through the result of FIG. 30, the cobalt removal rate according to the presence or absence of cells was confirmed. It was confirmed that a significantly excellent cobalt removal rate was shown in case of presence of the cells (w/ *C.vulgaris*) compared to the case when the cells were not present (w/o *C.vulgaris*) at all concentrations, and the specific measurement values of FIG. 14 were shown in Table 10 below.

**[Table 10]**

| | 300 mM | | 600 mM | | 900 mM | |
|---|---|---|---|---|---|---|
| | w/ *C.vulgaris* | w/o *C.vulgaris* | w/ *C.vulgaris* | w/o *C. vulgaris* | w/ *C. vulgaris* | w/o *C. vulgaris* |
| Co (ppm, µg/ml) | 5598.14 | 21531.30 | 12889.26 | 35803.51 | 14853.89 | 64582.16 |

### 11-2. Effect results on Sodium and Sulfate

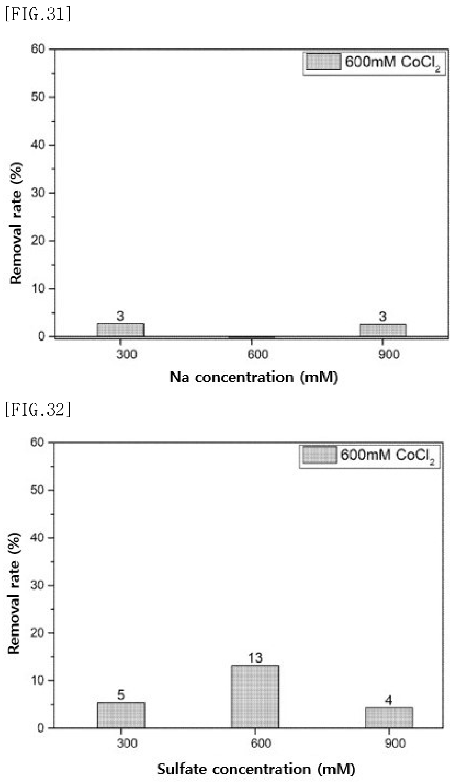

In order to measure the effect results on sodium and sulfate, experiments were performed by the same method as Example 11-1, but NaCl or potassium sulfate was further added to the Cornial tube by concentration (300, 600, 900mM). The concentration of Co was fixed at 600mM. The results were shown in FIG. 31 to FIG. 32.

As a result, through the result of FIG. 31, the cobalt removal rate according to the concentration of sodium was confirmed. It was confirmed that the cobalt removal rates of about 3% at 300 mM, about 0% at 600 mM, and about 3% at 900 mM were shown. In other words, it was confirmed that the constant correlation between the concentration of sodium and cobalt removal rate was not shown.

In addition, through the result of FIG. 32, the cobalt removal rate according to the concentration of sulfate was confirmed. It was confirmed that the cobalt removal rates of about 5% at 300 mM, about 13% at 600 mM and about 4% at 900 mM were shown. In other words, it was confirmed that the constant correlation between the concentration of sulfate and cobalt removal rate was not shown.

### Example 12. Preparation of cobalt battery solution

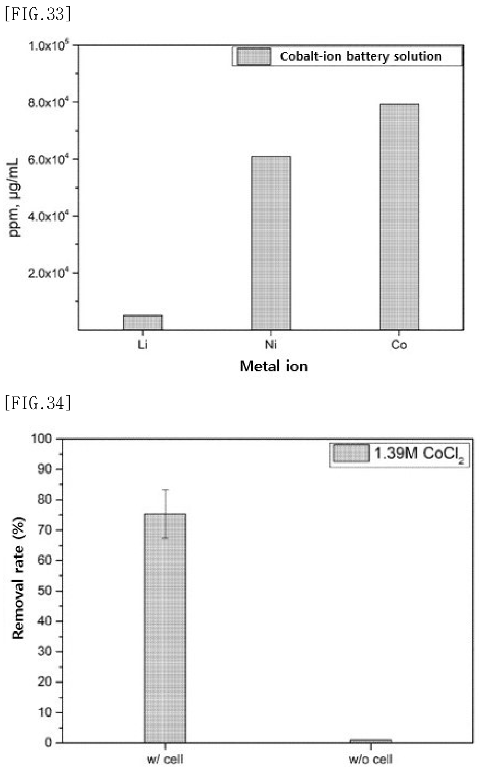

A cobalt battery solution was prepared by mixing LiCl₂ (sigma), NiCl₂ (sigma), CoCl₂ (sigma), MnCl (sigma), AlCl₃ (sigma), CuCl₂ (sigma), FeCl₂ (sigma), ClF (sigma), KH₂PO₄ (sigma) to DIW (deionized water). The composition of the prepared cobalt battery solution was analyzed and shown in Table 11 and FIG. 33 below.

**[Table 11]**

| **Elements** | **g per 1L** |
|---|---|
| Al | 5 |
| Co | 33 |
| Cu | 3 |
| Fe | 0.3 |
| Li | 4 |
| Mn | 11 |
| Ni | 26 |
| F | 4 |
| P | 1 |

As could be confirmed in Table 11, it was confirmed that it was Li 4%(1M), Ni 26%(1.09M), and Co 33%(1.39M).

### Example 13. Results of Cobalt (Co) biomineralization in lithium battery solution

### 13-1. Crystallization in cobalt battery solution

The Co crystallization result was confirmed in the cobalt battery solution, using the transformed *Chlorella vulgaris* (cell) in Example 3, the cobalt battery solution prepared in Example 12, sterilized MBM medium (KNO₃ 2.5mM, MgSO₄ 7H₂O 0.3mM, K₂HPO₄ 0.43mM, KH₂PO₄ 1.29mM, NaCl 0.43mM, CaCl₂*2H₂O 0.068mM, FeSO₄*7H₂O 0.1g, A5 metal mix ml/L), DIW (deionized water), white light, a stirrer, a 10ml Cornical tube, a centrifuge, and a 0.2uM syringe filter. The specific experimental method was as follows.

Fresh (log phase) cells of *Chlorella vulgaris* were cultured in the MBM medium by 6*10^7/ml. After harvesting 5ml of the cells collected in the medium, they were mixed with cobalt battery solution containing 5ml of 2.78M NiCl₂ using a 10ml Cornical tube, and finally, 10ml of 1.39M CoCl₂ solution was prepared. This was slowly stirred and reacted under white light for 16 hours. After 16 hours, at 3000rpm, centrifugation was performed for 3 minutes. The cells and crystals were filtered by a 0.2uM syringe filter by collecting the supernatant, and the collected solution was used for ICP analysis. Samples were ionized using inductively coupled plasma measured by loading the samples into ICP-OES and MS instruments, and then the corresponding ions were separated using a mass spectrometer and ICP analysis was performed. The result was shown in FIG. 34.

Through the result of FIG. 34, the cobalt removal rate according to the presence or absence of cells was confirmed. It was confirmed that cobalt was hardly removed when cells were not present (w/o Cell), but lithium of about 75% was removed when the cells were present (w/ Cell).

### 13-2. Crystallization according to Na, S in cobalt battery solution

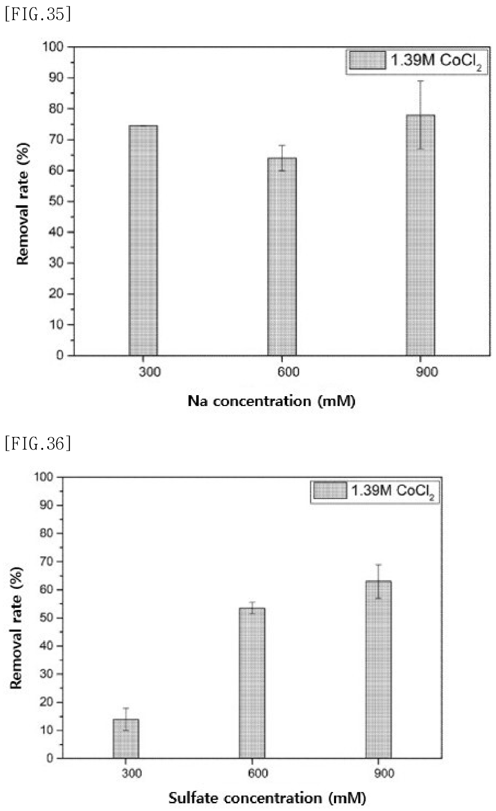

In order to measure the effect results on sodium and sulfate, experiments were performed by the same method as Example 13-1, but NaCl or potassium sulfate was further added to the Cornial tube by concentration (300, 600, 900mM). The results were shown in FIG. 35 to FIG. 36.

Through the result of FIG. 35, the cobalt removal rate according to the concentration of sodium was confirmed. It was confirmed that the cobalt removal rates of about 74% at 300 mM, about 64% at 600 mM, and about 77% at 900 mM were shown. In other words, it was confirmed that the constant correlation between the concentration of sodium and cobalt removal rate was not shown.

In addition, through the result of FIG. 36, the cobalt removal rate according to the concentration of sulfate was confirmed. It was confirmed that the cobalt removal rates of about 13% at 300 mM, about 53% at 600 mM and about 62% at 900 mM were shown. In other words, it was confirmed that the cobalt removal rate increased as the concentration of sulfate increased.

### Summary

The results of the lithium (Li) biomineralization results in the single LiCl₂ solution and lithium battery solution were summarized and shown in Tables 12 and 13 below.

**[Table 12]**

| **Concentration (LiCl,mM)** | **Removal rate (%)** | **Na Concentration (mM)** | **Δ Removal rate (600mM LiCl, %)** | **Sulfate Concentration (mM)** | **ΔRemoval rate (600mM LiCl, %)** |
|---|---|---|---|---|---|
| 300 | 71 | 300 | 13 | 300 | -12 |
| 600 | 94 | 600 | -3 | 600 | -1 |
| 900 | 28 | 900 | 17 | 900 | 13 |

**[Table 13]**

| **Concentration (LiCl ,mM)** | **Removal rate (%)** | **Na Concentration (mM)** | **Removal rate (1M LiCl, %)** | **Sulfate Concentration (mM)** | **Removal rate (1M LiCl, %)** |
|---|---|---|---|---|---|
| 300 | 52 | 300 | 56 | 300 | 33 |
| 600 | 73 | 600 | 68 | 600 | 45 |
| 900 | 52 | 900 | 37 | 900 | 75 |

The results of the nickel (Ni) biomineralization results in the single NiCl₂ solution and nickel battery solution were summarized and shown in Tables 14 and 15 below.

**[Table 14]**

| **Concentration (NiCl2,mM)** | **Removal rate (%)** | **Na Concentration (mM)** | **Δ Removal rate (600mM NiCl2, %)** | **Sulfate Concentration (mM)** | **Δ Removal rate (600mM NiCl2, %)** |
|---|---|---|---|---|---|
| 300 | 36 | 300 | 11 | 300 | 6 |
| 600 | 15 | 600 | 8 | 600 | 2 |
| 900 | 23 | 900 | 4 | 900 | 9 |

**[Table 15]**

| **Concentration (NiCl2,mM)** | **Removal rate (%)** | **Na Concentration (mM)** | **Removal rate (1M NiCl2, %)** | **Sulfate Concentration (mM)** | **Removal rate (1M NiCl2, %)** |
|---|---|---|---|---|---|
| 300 | 68 | 300 | 74 | 300 | 12 |
| 600 | 73 | 600 | 62 | 600 | 55 |
| 900 | 82 | 900 | 80 | 900 | 64 |

The results of the cobalt (Co) biomineralization results in the single CoCl₂ solution and cobalt battery solution were summarized and shown in Tables 16 and 17 below.

**[Table 16]**

| **Concentration (CoCl2,mM)** | **Removal rate (%)** | **Na Concentration (mM)** | **Δ Removal rate (600mM CoCl2, %)** | **Sulfate Concentration (mM)** | **Δ Removal rate (600mM CoCl2, %)** |
|---|---|---|---|---|---|
| 300 | 50 | 300 | 3 | 300 | 5 |
| 600 | 54 | 600 | 0 | 600 | 13 |
| 900 | 30 | 900 | 3 | 900 | 4 |

**[Table 17]**

| **Concentration (CoCl2,mM)** | **Removal rate (%)** | **Na Concentration (mM)** | **Removal rate (1.39M CoCl2, %)** | **Sulfate Concentration (mM)** | **Removal rate (1.39M CoCl2, %)** |
|---|---|---|---|---|---|
| 300 | 69 | 300 | 74 | 300 | 13 |
| 600 | 67 | 600 | 64 | 600 | 53 |
| 900 | 73 | 900 | 77 | 900 | 62 |

### INDUSTRIAL AVAILABILITY

The present invention relates to a composition for leaching lithium, nickel or cobalt, and a method for leaching lithium, nickel or cobalt, using a *Chlorella vulgaris* strain.

## Claims

1. A composition for leaching lithium, nickel or cobalt, comprising at least one kind selected from the group consisting of a *Chlorella vulgaris* strain, a culture of the strain, a concentrate of the culture, and a dried product of the culture.

2. The composition for leaching lithium, nickel or cobalt according to claim 1, wherein the strain is transformed with a vector comprising the following:
a promoter of the *Coccomyxa* C-169 ribulose-1,5-bisphosphate carboxylase/oxygenase (RuBisCo) small subunit 2 (rbcS2) gene;
a nucleotide sequence encoding beta-type carbonic anhydrase of Coccomyxa subellipsoidea C-169; and
a terminator sequence of the *Coccomyxa* C-169 rbcS2 gene.

3. The composition for leaching lithium, nickel or cobalt according to claim 2, wherein the promoter comprises the nucleotide sequence of SEQ ID NO: 2.

4. The composition for leaching lithium, nickel or cobalt according to claim 2, wherein the nucleotide sequence encoding beta-type carbonic anhydrase of Coccomyxa subellipsoidea C-169 is operatively linked to the promoter.

5. The composition for leaching lithium, nickel or cobalt according to claim 2, wherein the, the terminator sequence comprises the nucleotide sequence of SEQ ID NO: 3.

6. The composition for leaching lithium, nickel or cobalt according to claim 2, wherein the vector further comprises an antibiotic resistance gene as a selectable marker.

7. The composition for leaching lithium, nickel or cobalt according to claim 6, wherein the antibiotic is at least one kind selected from the group consisting of spectinomycin, paromomycin, ampicillin, zeocin and bleomycin.

8. The composition for leaching lithium, nickel or cobalt according to claim 2, wherein the vector further comprises a gene encoding a reporter molecule.

9. The composition for leaching lithium, nickel or cobalt according to claim 8, wherein the reporter molecule is at least one kind selected from the group consisting of a growth-promoting protein, a fluorescent protein and a hydrolytic enzyme.

10. The composition for leaching lithium, nickel or cobalt according to claim 9, wherein the fluorescent protein is luciferase.

11. The composition for leaching lithium, nickel or cobalt according to claim 9, wherein the hydrolytic enzyme is β-glucuronidase.

12. The composition for leaching lithium, nickel or cobalt according to claim 2, wherein the vector is for microalgal transformation using gold particles bombardment.

13. A method for leaching lithium, nickel or cobalt through bioleaching, comprising
a mixing step of mixing at least one kind selected from the group consisting of a *Chlorella vulgaris* strain, a culture of the strain, a concentrate of the culture, and a dried product of the culture, with a substance containing lithium, and
a leaching step of leaching lithium for a predetermined period of time.

14. The method for leaching lithium, nickel or cobalt according to claim 13, wherein the method for leaching lithium, nickel or cobalt comprises the following transforming step before the mixing step, and the *Chlorella vulgaris* strain of the mixing step is a transformed *Chlorella vulgaris* strain:
a transforming step of introducing a vector comprising a promoter of the *Coccomyxa* C-169 ribulose-1,5-bisphosphate carboxylase/oxygenase (RuBisCo) small subunit 2 (rbcS2) gene, a nucleotide sequence encoding beta-type carbonic anhydrase of Coccomyxa subellipsoidea C-169, and a terminator sequence of the *Coccomyxa* C-169 rbcS2 gene, into *Chlorella vulgaris.*

15. The method for leaching lithium, nickel or cobalt according to claim 14, wherein the promoter comprises the nucleotide sequence of SEQ ID NO: 2.

16. The method for leaching lithium, nickel or cobalt according to claim 14, wherein the nucleotide sequence encoding beta-type carbonic anhydrase of Coccomyxa subellipsoidea C-169 is operatively linked to the promoter.

17. The method for leaching lithium, nickel or cobalt according to claim 14, wherein the terminator sequence comprises the nucleotide sequence of SEQ ID NO: 3.

18. The method for leaching lithium, nickel or cobalt according to claim 14, wherein the vector further comprises an antibiotic resistance gene as a selectable marker.

19. The method for leaching lithium, nickel or cobalt according to claim 18, wherein the antibiotic is at least one kind selected from the group consisting of spectinomycin, paromomycin, ampicillin, zeocin and bleomycin.

20. The method for leaching lithium, nickel or cobalt according to claim 14, wherein the vector further comprises a gene encoding a reporter molecule.

21. The method for leaching lithium, nickel or cobalt according to claim 20, wherein the reporter molecule is at least one kind selected from the group consisting of a growth-promoting protein, a fluorescent protein and a hydrolytic enzyme.

22. The method for leaching lithium, nickel or cobalt according to claim 21, wherein the fluorescent protein is luciferase.

23. The method for leaching lithium, nickel or cobalt according to claim 21, wherein the hydrolytic enzyme is β-glucuronidase.

24. The method for leaching lithium, nickel or cobalt according to claim 14, wherein the vector is for microalgal transformation using gold particles bombardment.

25. The method for leaching lithium, nickel or cobalt according to claim 14, wherein the transforming step is performed using gold particles bombardment.

26. The method for leaching lithium, nickel or cobalt according to claim 14, wherein the cell stage of the transforming step is a log phase.

27. The method for leaching lithium, nickel or cobalt according to claim 26, wherein the log phase has an OD686 value of 0.4 to 0.6.

28. The method for leaching lithium, nickel or cobalt according to claim 14, wherein the cell density of the transforming step is 5.0*10⁶ to 8.0*10⁷ per 60mm Diameter.

29. The method for leaching lithium, nickel or cobalt according to claim 14, wherein the vacuum of the transforming step is 27.0 to 29.0 inchs Hg.
